Europäisches Patentamt

European Patent Office

Office européen des brevets

(11) Publication number: 0 242 723
A1

# EUROPEAN PATENT APPLICATION

(21) Application number: 87105279.1

(22) Date of filing: 09.04.87

(51) Int. Cl.4: A61B 3/02

(30) Priority: 10.04.86 US 850281

(43) Date of publication of application:
28.10.87 Bulletin 87/44

(84) Designated Contracting States:
DE FR GB IT SE

(71) Applicant: TECHNA VISION, INC.
11489 Woodside Avenue
Santee California 92017(US)

(72) Inventor: Brown, William E.
9313 Viento Fuerte
La Mesa California(US)
Inventor: Houghton, George K.
6172 Syracuse Lane
San Diego California 92122(US)
Inventor: Rydeen, Clarence F.
25333 Poderio Dr.
Ramona California 92065(US)
Inventor: LaSalle, Richard A.
241 Old Dirt Road
Tallahassee Florida 32301(US)
Inventor: Kelley, James T.
13516 Silver Lake Drive
Poway California 92064(US)
Inventor: Forehand, Joseph M.
6243 E. Lake Dr.
San Diego California 92119(US)
Inventor: Massie, Norbert A.
9707 Caminito Joven
San Diego California 92131(US)

(74) Representative: Altenburg, Udo, Dipl.-Phys. et
al
Patent- und Rechtsanwälte
Bardehle-Pagenberg-Dost-Altenburg-Frohwi-
tter & Partner Postfach 86 06 20
D-8000 München 86(DE)

(54) Optical-mechanical system for an automated perimeter.

(57) Disclosed is an optical-mechanical system (12) for an automated perimeter which projects a focused spot of light of varying brightness, size and color onto the white, inside surface of a hemispherical bowl (14) uniformly illuminated at about 31.55 APS. It includes a head positioning assembly (16) for automatically positioning patient's head so that the eye being tested will be correctly aligned with a fixation target (14b) at the apex of the bowl (14).

Cat's eye (74) and periscope mirror assemblies (78) are employed to move the spot to selected positions by stepping motors that are quiet and allow for accurate control of positioning and smooth movement from one position to the next.

FIG.2

## OPTICAL-MECHANICAL SYSTEM FOR AUTOMATED PERIMETER

### BACKGROUND OF THE INVENTION

#### 1. Field of the Invention

This invention relates to an automated perimeter, and particularly, to the optical-mechanical system for an automated perimeter.

#### 2. Background Discussion

Visual field testing is conducted to determine the sensitivity of the retina to a light stimulus. This test is used, for example, to detect the early onset of glaucoma. For visual field testing, the patient's eye should be held in a steady position by having him or her look at a specific point - the "fixation target" or "point of fixation." At some other place within the field of vision is presented a spot stimulus of light and the patient communicates whether or not he or she can see it.

With a typical projection perimeter, i.e., the Goldmann perimeter, this would be a projected spot of white light of a selected size of the spot and background of a certain standard intensity. Keeping the size of the spot and the background illumination the same, and starting with the spot too dim to see, the examiner successively increases the spot intensity until the patient reports that he or she can see it. The dimmest stimulus that can be seen is recorded as the threshold stimulus for that location in the field. Both static and kinetic tests may be conducted. Various types of automated perimeters are described in U. S. Patent Nos. 3,718,386; 4,045,130; 4,146,311; and 4,260,227.

The field of vision testing traditionally has followed standards promulgated by Dr. Goldmann. The Goldmann standards are discussed in depth in "Visual Fields - A Basis for Efficient Investigation" by C. H. Bedwell, Published by Butterworth Scientific. Goldmann standards govern the brightness of the light spot, the size of the spot, spot position on the background, and the number of test performed. Color may also be controlled. All theses test conditions may be varied, depending upon the extent to which a particular eye examination is to be conducted. For example, the examiner may only wish to conduct a screening test to look for gross defects. Or, the examiner may wish to examine in detail all or a portion of the patient's retina. To accomplish this on a fully automated basis presents many problems.

First, the brightness of the light must be variable in a controlled fashion. Second, spot size and color must also be changed. Third, it is necessary to move the light spot to different locations and keep the spot in focus using a mechanical system which is relatively silent. If the mechanical system is noisy, this will not only distract the patient, but may give him a clue as to when the spot is presented. Thus, the test would be invalid. Forth, the background illumination must be constant and uniform throughout the field of vision. Fifth, the patient must fixate his or her eye during testing.

### MAJOR FEATURES OF THE INVENTION

The problems discussed above have been obviated by the present invention which provides an improved optical-mechanical system for an automated perimeter. The perimeter using the optical-mechanical system of this invention will provide test data comparable to that performed manually in accordance with the Goldmann procedures, with increased accuracy, better repetition, and increased patient comfort.

There are several features of this invention, no single one of which is solely responsible for its desirable attributes. Without limiting the scope of this invention, as expressed by the claims, its more prominant features will now be discussed briefly. After considering this discussion, and particularly after reading the section of this application entitled "DETAILED DESCRIPTION OF PREFERRED EMBODIMENTS, one will understand how the features of this invention provide the advantages of this invention.

First, movable mirrors are used that are adjustable so that the light will be focused even though the size of the projected light stimulus or spot may be varied and moved to different position. This is accomplished using a pair of mirrors which are moved together in unison along a horizontal path and are disposed at a 90° angle with respect to each other. Such mirrors are flat and have been nicknamed "cat's eye" mirrors. Using such movable "cat's eye" mirrors enables the ratio of distances between (1) the object (the light entrance opening) and the lens and (2) the lens and the image (the spot) to be maintained constant even though the spot is moved to different positions. Thus, the spot is maintained in focus.

Second, the movement of the mirrors is accomplished by stepping motors which move the light spot in short increments or steps. As viewed from the patient's eye looking at the inside surface of the bowl on which the light spot is projected, the light spot will be moved by the stepping motors one sixtieth (1/60) of a degree or less by microstepping the motors. Microstepping of the light spot gives the impression to the patient that the spot is moving continuously rather than in steps. Microstepping is a well known technique, and Compu-Motors are a suitable brand of motor that employs microstepping. Such motors are operated by computers in accordance with a predetermined program. Care is taken so that the mounting of the stepping motors is such that vibrations are minimized, thus minimizing noise.

Third, it is necessary that the patient fixate his or her eye at the center of the bowl on which the light spot is being projected. Fixation means are employed which allow the patient to look at the center or apex of the bowl at which a light is located that serves as the target on which the patient may fixate his or her eye. Preferably, a low light level video camera, e.g. a Nuvicon camera, is located behind a beam splitter that reflects an image of the light source towards the patient's eye. The video camera is behind the beam splitter and monitors the patient's eye and displays an image of the eye on a monitor screen. Thus, if the patient moves his eye, the TV camera will display this movement on the monitor screen and the examiner may invalidate the test based on this information.

Fourth, the bowl should be illuminated uniformly. We accomplish this by providing a light source mounted at the lower front portion of the bowl member having means for selectively distributing the amount of light over the internal surface so that the light is uniform. A light bulb holder having an open mouth is provided so that the filament of the light bulb is just at or slightly below the open mouth. This will eliminate glare being projected onto the surface of the bowl due to the high concentration of light eminating from the filament.

The preferred embodiments of the invention illustrating all its features will now be discussed in detail. These embodiments show the invention being used to conduct the standard Goldmann field of vision test. Because of the versatility of this invention, the examiner may now replicate the manual procedures of the Goldmann test and obtain data presented in a format similar to that obtained using the manual techniques. Using this invention, the examiner may now collect data quicker, with reduced errors, greater patient comfort, and with enhanced repeatability.

## BRIEF DESCRIPTION OF THE DRAWING

The optical-mechanical system for an automated perimeter of this invention is illustrated in the drawing in which

FIG. 1 is a perspective view of an automated perimeter using the optical-mechanical system of this invention.

FIG. 2 is a side elevational view of the primeter shown in FIG. 1, with the housing removed and partially in cross section.

FIG. 3 is a front elevational view of the perimeter shown in FIG. 2 with the housing removed.

FIG. 4 is a schematic plan view of one embodiment of the light spot projection system used in the optical mechanical system of this invention.

FIG. 5 is a schematic side view of the light spot projection system shown in FIG. 4.

FIG. 6 is a schematic view of one embodiment of the eye fixation device used in the optical mechanical system of this invention.

FIG. 7 is a schematic view of an alternate embodiment of an eye fixation device.

FIG. 8 is a side elevational view of the light spot control assembly.

FIG. 9 is a cross-sectional view taken along 9-9 of FIG. 8.

FIG. 10 is a plan view of the combination shutter/spot size/color wheel used in the light spot control assembly shown in FIG. 8.

FIG. 11 is the filter wheel used in the light spot control assembly shown in Fig. 8.

FIG. 12 is a plan view of an alternate embodiment of the light spot projection system.

FIG. 13 is a schematic view of the optical path used in the light spot projection system shown in FIG. 12.

FIG. 14 is a rear elevational view of the periscope mirror assembly for the light spot projection system shown in FIG. 12.

FIG. 15 is a side elevationa view of the periscope mirror assembly shown in FIG. 14.

FIG. 16 is a plan view of the periscope mirror assembly shown in FIG. 14.

FIG. 17 is a cross-sectional view of the bowl illumination assembly taken along line 17-17 of FIG. 5.

FIG. 18 is a cross-sectional view of the bowl illumination assembly taken along line 18-18 of FIG. 5.

FIG. 19 is a cross-sectional view of the bulb holder shown in FIG. 17 taken along line 19-19 of FIG. 17.

FIG. 20 is a perspective view of the bowl illumination assembly.

## DESCRIPTION OF THE PREFERRED EMBODIMENTS

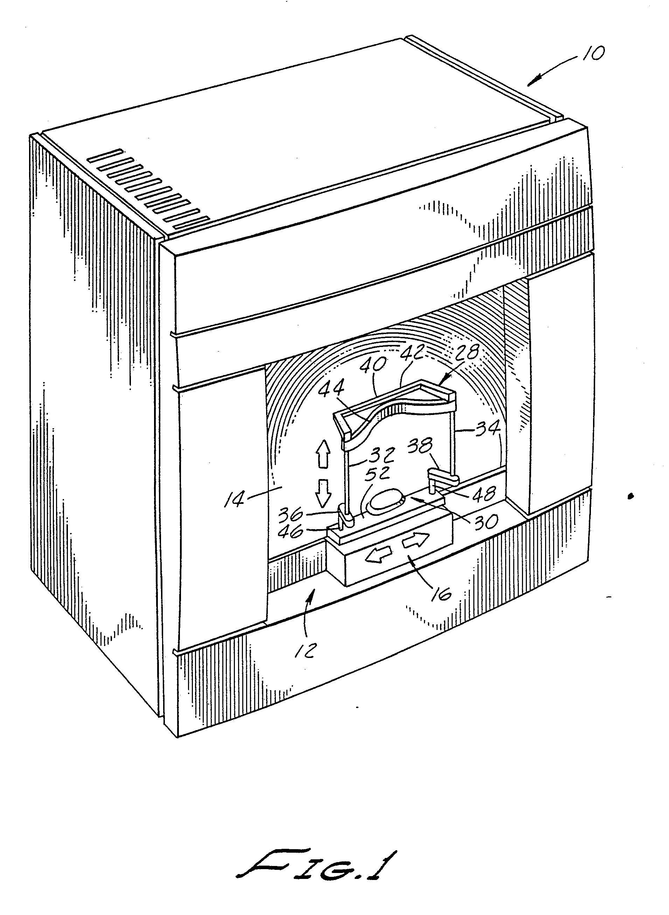

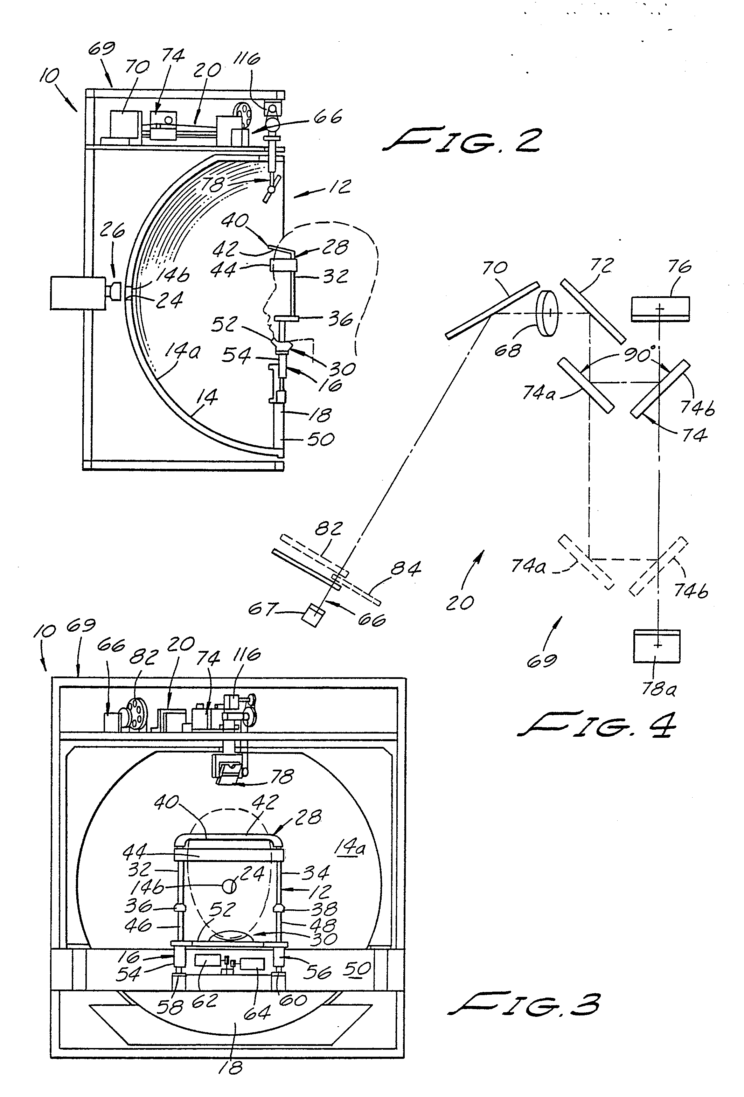

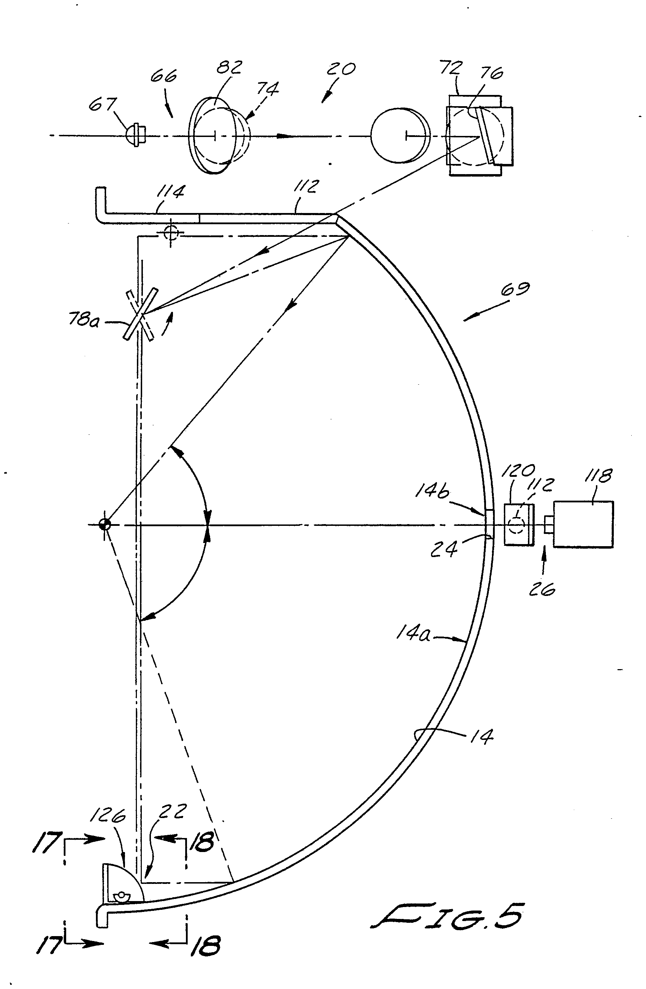

FIGS. 1 through 5 show an automated perimeter 10 using the optical-mechanical system 12 of the present invention. This system 12 includes a bowl 14, a head positioning assembly 16 at the lower front portion 18 of the open side of the bowl, the spot projection system 20 mounted generally about the bowl, and the bowl illumination assembly 22 mounted at the lower front portion of the bowl beneath the head positioning assembly.

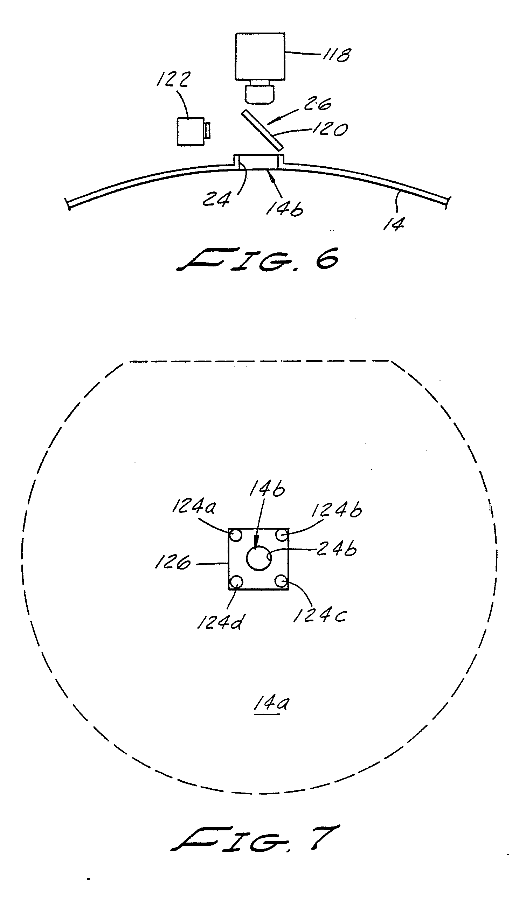

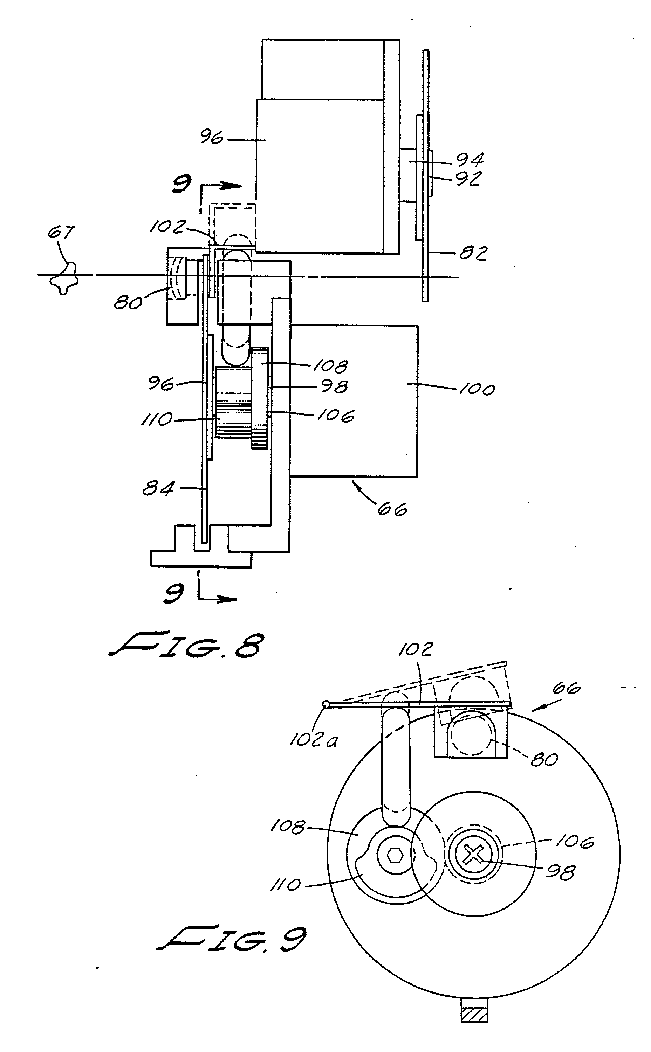

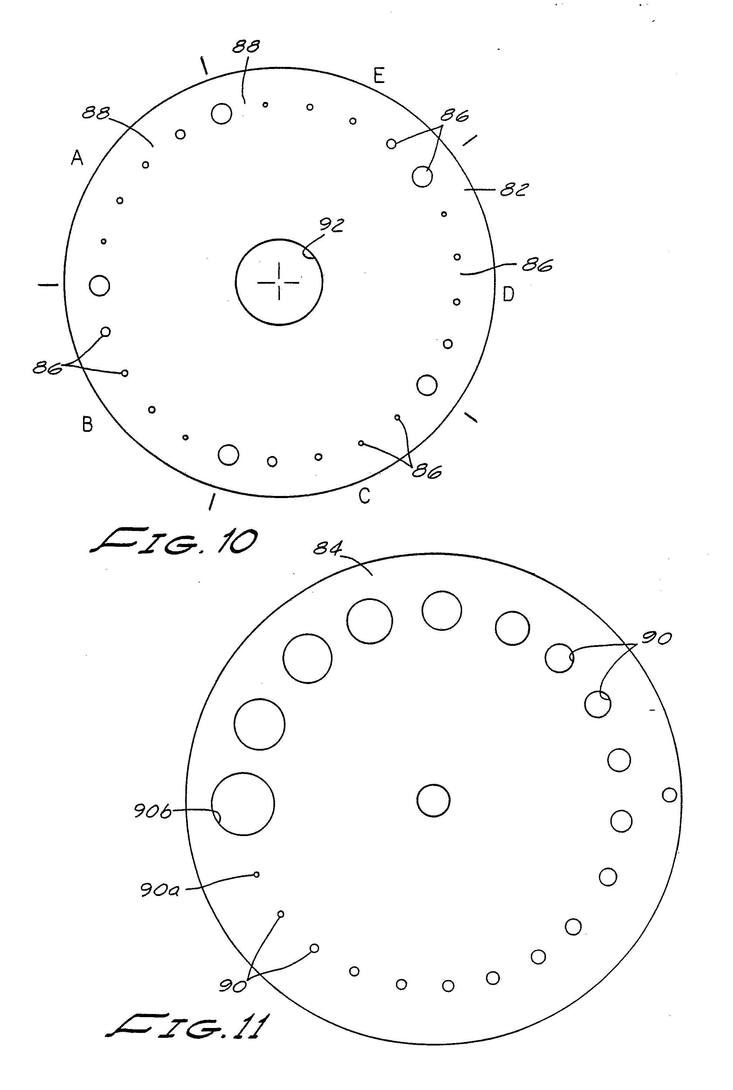

The bowl 14 is a hemispherical member having a white interior surface 14a on which a light spot is projected by the spot projection system 20. At the apex 14b of the bowl is an opening 24 approximately three-quarters of an inch in diameter. As discussed subsequently, an eye fixation device 26 (FIG. 6) is located near the apex 14b of this bowl. The bowl 14 has a diameter of 33 centimeters. This is the standard promulgated by Dr. Goldmann. The depth of the bowl is approximately one-half the bowl's diameter.

The head positioning assembly 16 is designed to move the patient's head into a position where the eye being tested is aligned directly opposite the opening 24 at the apex 14b of the bowl 14. With the patient in this position, his or her eye can detect light stimulation upwardly through an angle of 50°, downwardly through an agle of 76°, and side to side in either direction through an angle of 80°. The area inscribed by these angles on the interior surface 14a of the bowl is the field onto which the light spot is projected by the spot projection system 20.

The head positioning assembly 16 has two major sections: a head rest section 28 and a chin rest section 30. The head rest section 28 includes a first pair of posts 32 and 34 having their lower ends fixidly mounted in vertical alignment on the outer ends of a pair of arms 36 and 38 that extend outwardly from the bowl 14 and flair away from each other. A crosspiece 40 having an inwardly projecting element 42 is secured to the upper end of these posts 32 and 34. A head strap 44 of flexible plastic material extends between the posts 32 and 34 and has its respective ends attached to the posts. There may also be attached to the crosspiece 40 a holder (not shown) for corrective lenses in case the person being tested has a large refractive error. The headrest section 28 also includes a second, lower pair of posts 46 and 48 which have their upper ends attached securely to the arms 36 and 38 and their lower ends secured to the chin rest section 30.

The chin rest section 30 is mounted on horizontal tracks (not shown) carried on the lower front panel 50. This allows the chin rest section 30 to move reciprocally in a horizontal direction. The chin rest section 30 includes a support member 52 which extends between the arms 46 and 48 and carries at its opposed ends bearing members 54 and 56 which receive the arms 46 and 48 of the head rest section. These bearing members 54 and 56 are mounted on screw drives 58 and 60 which, when activated, lower or raise the head positioning assembly 16. A pair of motors 62 and 64 are carried on the panel 50 which drive pulley systems (not shown) to either raise or lower the head positioning assembly or movement sideways in either direction. The head positioning assembly 16 thus allows the examiner to automatically adjust the position of the patient's head by actuation of the drive motors 62 and 64.

As shown in FIG. 4 the spot projection system 20 includes a light source assembly 66, a lens 68, and mirror assembly 69 including two stationary mirrors 70 and 72 on opposite sides of the lens, a pair of opposed mirrors 74 and 74b (herein the cat's eye mirror assembly 74) mounted to move together in unison to and fro along a horizontal path, another stationary mirror 76 and a periscope mirror assembly 78. The angle between the mirrors 74a and 74b of the cat's eye assembly 74 are such that their sum is 90°.

The purpose of the spot projection system 20 is five-fold. First, it focuses the light spot on the internal surface 14a of the bowl 14. Second, it controls the size of the light spot so that it corresponds to one of the Goldmann standard sizes. Third, it moves the light spot to different positions on the internal surface 14a of the bowl 14. Fourth, it changes the color of the light spot. And fifth, it changes the brightness of the light spot.

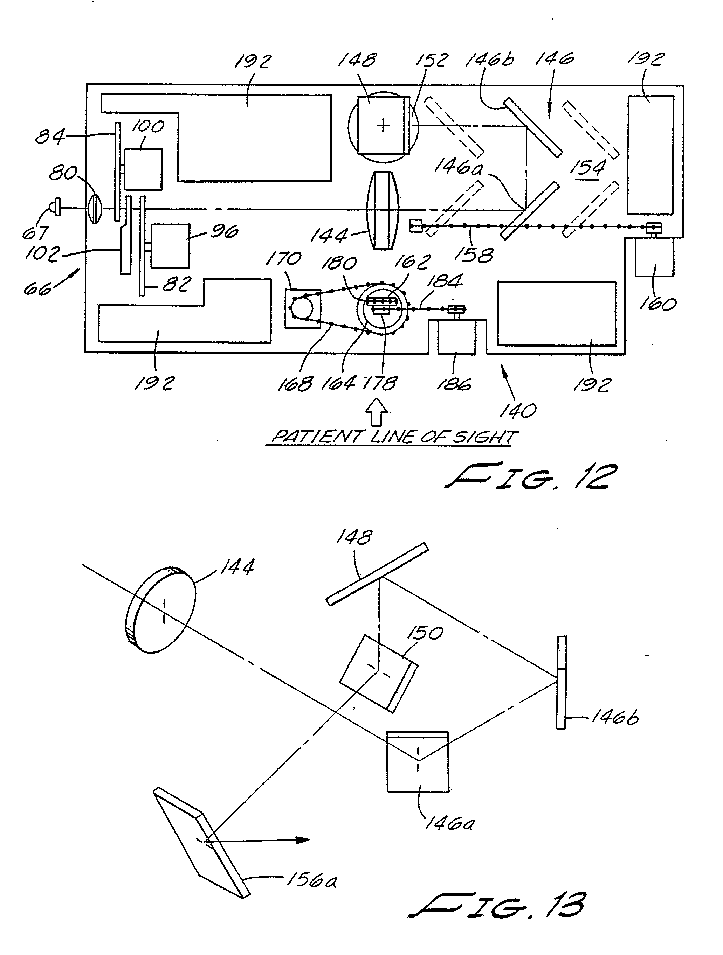

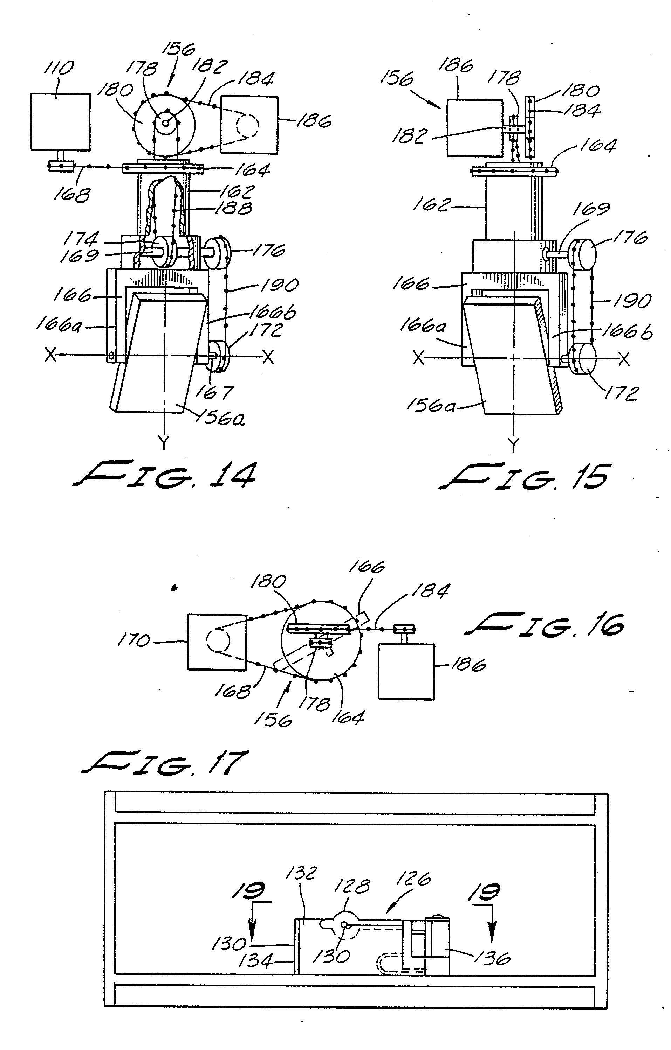

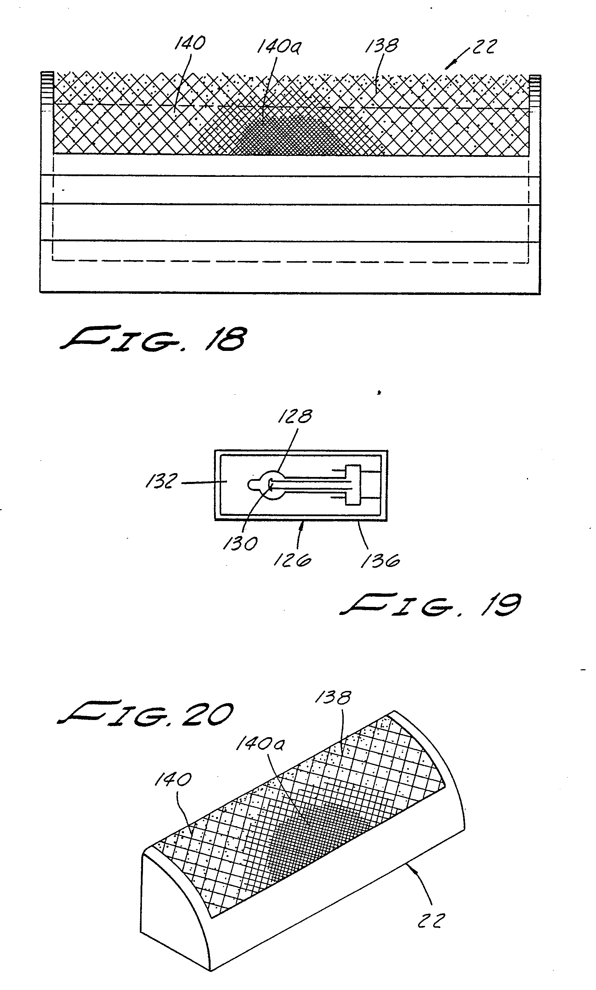

As shown in FIGS. 9 through 11, the light source assembly 66 includes a halogen bulb 67, a lens 80, a combination shutter/spot size or wheel 82, and filter wheel 84. The bulb 67 contains a halogen gas such as iodine and employs a tungsten filament (not shown). It is desirable at the start of the day to turn on the bulb and leave it on until all examinations have been completed for the day using a low current level when not in use. This will minimize the changes of brightness in the bulb 67. A constant brightness of the bulb is necessary to properly conduct the test. By maintaining the intensity of the bulb constant, and the color temperature of the bulb constant at a high color temperature, for example of 2900°, the light emitted from the bulb will have constant brightness and its spectral distribution will be within the significant portion of the visible range. The brightness of the light preferably is of sufficient intensity so that when it passes through the various filters and reflects off the mirrors, it will provide a light spot on the inside surface 14a of the bowl 14 ranging between 10 and 10,000 apostilbs (ASP).

As shown in FIG. 10, the combination wheel 82 has a plurality of holes 86 of different sizes. It consists of a plate divided up into five perimeter sections A, B, C, D and E with an equal number of holes 86 in each of the sections along the perimeter of the wheel. As will be explained in greater detail below, these holes 86 control the size of the spot. The spacings between the holes is such that there is a plate segment 88 between each hole to block the light coming from the bulb 67 when the wheel 82 is positioned with the segment 88 between the bulb 67 and lens 68. The five different sections A, B, C, D and E correspond to different colors: red, blue, green, yellow and clear. These colors are provided by color filters (not shown) which are inserted into the holes. For example, a red filter is in each hole in Section A, a blue in Section B, etc. The combination wheel 82 is mounted as shown in FIG. 8 so that its perimeter is disposed in the beam of light coming from the lens 80. The hub 92 of the wheel 84 is connected to a shaft 94 that is rotated by a motor 96. The size of the light spot being projected onto the inside surface 14a of the bowl 14 is determined by which hole 86 is moved into the path of the beam of light. Each section A through E has five holes in a series with the diameters of the holes increasing in size. Spot size is increased by moving larger diameter holes 86 into the path of the light and decreased by moving smaller diameter holes into the light path. Moving a plate segment 88 into the light path blocks the light, preventing the spot from being projected onto the surface 14a. The color of the spot is governed by which section of the wheel 82 is positioned in the light path.

The filter wheel 84 controls the brightness of the light spot. It includes a series of holes 90 about the wheel's perimeter varying incrementally in diameter from a very small hole 90a to a relatively large hole 90b. This wheel 84 is mounted so that its perimeter is disposed in the path of light. The hub 96 of the wheel 84 is mounted on a shaft 98 that is rotated by a motor 100. Rotation of the shaft 98 by the motor 100 brings holes 90 of different sizes into position adjacent the lens 80. Large holes allows more light to pass than smaller holes, so when the brightness of the light spot is to be increased, a larger hole is moved into position until the desired brightness is attained. A neutral density filter element 102 is used to double the number of brightness levels.

The neutral density filter element 102 is pivotly mounted at one end 102a, and raised out of the light path or lowered into the light path by means of a cam actuated plunger 104. A gear 106 on the shaft 98 meshes with a gear 108 carried by a cam 110. The ratio of the gears 106 and 108 is such that with the shaft 98 rotating through one cycle (360°), the plunger will remain in the down position shown in solid lines. Thus, the filter element 102 is filtering the light. The wheel 84, by rotating back and forth within this one cycle, will bring any selected one of the holes into the light path. This will provide twenty different brightness levels. By rotating the shaft 98 through a second complete second half cycle (720°), the plunger 104 is elevated as shown in dotted lines, lifting the filter element out of the light path. By rotating within this second complete cycle, this provides for an additional 20 brightness levels.

The mirror assembly 69 is designed to project the light spot onto different areas of the internal surface 14a of the bowl 14 while keeping it in focus. This is accomplished by maintaining the distance between the opening 86 in the combination wheel 82 and the lens 68 equal to the distance between the lens 68 and the area (A) where the light strikes the internal surface 14a. The periscope mirror 78 assembly 78 includes a flat mirror 78a that is mounted to rotate about both its horizontal and vertical axis. When the light is reflected off this mirror 78a, it will strike different areas (A) depending on the relative position of the mirror 78a. The distance between the surface of the mirror 78a and the area (A) then varies. To compensate for this variation, the cat's eye mirror 74 is moved to different positions to either shorten or lengthen the distance between the mirror 72 and the mirror 78a so that the total distance between the lens 68 and the area (A) equals the distance between the hole 86 and the lens 68.

The path the light follows as it leaves the hole 86 is diagramed in FIGS. 4 and 5. First, the light strikes the flat, stationary mirror 70 and passes through the lens 68. The mirror 72 on the other side of the lens 68 is also stationary and it directs the light passing through the lens onto the first mirror 74a of the cat's eye assembly 74. This mirror 74a projects the light onto the second mirror 74b of the cat's eye assembly and back to the stationary mirror 76 which projects the light through an opening 112 in the floor 114 on which the mirror assembly 69 sets to the mirror 78a of the periscope assembly 78. The mirror 78a of the periscope assembly then projects the light spot onto the internal surface 14a of the bowl 14.

The mirrors 74a and 74b are moved to the different positions by a stepping motor (not shown). A stepping motor 116 is used to move the mirror 78 also. If the steps are very large, this will be detected by the patient's eye as he will see the light spot moved from one point to another in discreet steps. If the steps are very small, however, the patient will not detect the stepping motion. In accordance with one feature of this invention, we use a stepping motor which moves these mirrors 74a, 74b, and 78a in short steps of less than about 1/60°. The use of stepping motors is highly desirable because the location of the spot can always be determined by simply counting the steps in any direction that it is being moved with the spot starting from a known position. Since the steps are precisely controlled and do not vary, spot position can be accurately determined. This is necessary in order to perform an accurate test. Because stepping motors are used, complex feedback control systems are thus eliminated.

FIGS. 5 and 6 illustrate the eye fixation device 26 of this invention. In accordance with this feature of the invention, there is located behind the opening 24 in the apex 14b of the bowl 14 a video camera 118. This is optional, but desirable. Disposed between the opening 24 and the lens of the camera 118 is a transmission mirror 120 which enables light to pass through it and into the lens of the video camera. Off to one side of this mirror is a light emitting diode 122. The light from this diode 122 will pass through the mirror except for approximately 10 percent, which will be reflected into the interior bowl through the opening 24. This light provides a target on which the patient can fixate. With the patient's pupil fixating on the image of light emitting diode, the video camera 118 will collect the light from the eye, which passes through the transmission mirror 120, and project an image of the eye on to a CRT (not shown). This enables the examiner to monitor the eye or record a picture of the eye on video tape.

FIG. 7 shows an alternate embodiment of the eye fixation device. Some patients have the central portion of their retina defective and will not be able therefore to fixate on a light image at the center of the bowl as depicted in FIGS. 5 and 6. In these cases, the opening 24 will be plugged with a suitable plug member 24b and four diodes 124a, 124b, 124c, and 124d equally spaced from the apex 14b at the corners of an imaginary square 126 provide the means for eye fixation. These light emitting diodes 124a-124d preferably are coated with a paint which will obscure them when they are not emitting light but will be of sufficient density to enable light to pass through this opaque barrier when the diodes are turned on. When a patient has a central retina defect, he will be able to see the four lite diodes, and by looking at the center of these four lite diodes, he will fixate and enable the examiner to conduct the field of vision test.

### Bowl Illumination

Another feature of this invention is bowl illumination. Uniform bowl illumination is highly desirable, and in some instances, may be critical for proper testing. Uniform bulb illumination is accomplished by means of a light source 126 which is located in the lower front central portion 18 of the bowl member 14. This light source 126 includes a halogen bulb 128 with tungston filament 130 of matching color temperature with the bulb used in the light source assembly 66. The bulb 128 is mounted within a cylindrical aluminum tubing 130 with the upper section cut away to provide an open mouth 132. The front end 134 of the tube is closed off and the rear end is mounted to a ceramic post-assembly 136 with the electrical leads for the bulb fastened to the assembly 136 and being connected to the filament 130. The filament 130 is positioned carefully relative to the mouth 132. The filament 130 runs crosswise to the length of the bulb, as illustrated in FIG. 19. It is about equal distance from the two ends of the tubing and position so that it is just slightly below the open mouth 132. This positioning is very important, otherwise the image of the filament is projected into the bowl 14 to provide a hot spot. The light source 126 is mounted in a chamber 138 which carries on its surface a filter element 140. The center portion 140a of the filter element 140 has more dots, because of the need to block more light to attain uniformity. The filter element 140 carefully designed so that light is blocked in such a fashion that the intensity of the light throughout the entire surface 14a of the bowl 14 is approximately 31.5 APS ± 5%.

### Alternate Mirror Assembly

FIGS. 12 through 16 illustrate an alternate mirror assembly 140 for use in the optical-mechanical system 12 of this invention. This assembly 140 is disposed so that its longitudinal axis is at a right angle relative to the line of sight of the patient being tested. This assembly 140 is designed to minimize noise, and it includes a light source assembly which is identical to assembly 66 and has been given the same number, a lens 144, a cat's eye mirror assembly 146 including mirrors 146a and 146b disposed relative to each other at an angle of 90°, a stationary flat mirror 148, a second stationary flat mirror 150 disposed below an opening 152 in the floor 154 of the assembly, and a periscope mirror assembly 156, including a flat movable mirror 156a. The optical path of the light eminating from the assembly 142.

The cat's eye mirror assembly 146 is mounted to move along a horizontal path and tracks (not shown). A pulley 158 attached to this assembly 146 is driven by a stepping motor 160 to move the assembly back and forth as required to compensate for the position of the light spot. The dotted line positions of the mirrors 146a and 146b shown the two extreme position of the cat's eye mirror assembly 146.

The periscope mirror assembly 156 is best illustrated in FIGS. 14 through 16. It includes a hollow vertical member 162 having an open top with a gear 164 about its edge. A U-shaped bracket 166 is secured to the bottom of the member 162. The mirror 156a is mounted on a shaft 167 carried between the legs 166a and 166b of the bracket 166 so that it may tilt to and fro about the horizontal axis X. The member 162 is carried within the floor 154 so that it may rotate, thus rotating the mirror 156a about the vertical axis Y. This member 162 is rotated by a belt 168 that is wrapped around the gear 164 and driven by a stepping motor 170. The mirror 156a is rotated about the horizontal axis (X) by rotation of the shaft 167.

The drive assembly for the shaft 167 includes a gear 172 mounted to the end of the shaft 167, a pair of gears 174 and 176 carried on a shaft 169 that is mounted in the member 162 above the bracket 166, the gears 178 and 180. The gears 178 and 180 are mounted on a common shaft 182 (FIG. 15). The gear 180 is rotated either clockwise or counterclockwise by a drive belt 184 from a stepping motor 186. This causes the gear 178 to rotate, and this rotational movement is transmitted through a drive belt 188 wrapped around the gears 178 and 174 to the shaft 169. Rotation of the shaft 169 rotates the gear 176 and this rotational movement is transmitted by a drive belt 190 wrapped around the gears 176 and 172 to turn the shaft 167. Thus, the mirror 156a is rotated about the horizontal axis (X) in response to the stepping motor 186 in incremental movements of less than 1/60°. Rotation of the hollow member 162 rotates the mirror about the vertical axis (Y) and twists the belt 188. This twisting does not interfere with the operation of the periscope assembly 156.

Because the motors 110 and 186 are not mounted directly on the member 162, their vibrations are dampened. This minimizes any jittering movement of the light spot as it travels across the internal surface 14a of the bowl 14. To further minimize vibrations, the mirror assembly 140 includes damping member 192 near its corners. These members are hollow aluminum cubes containing sand, with the particles of said bonded together by a rubber type cement.

## OPERATION

The automated perimeter 10 using the optical mechanical system 12 of this invention provides field of vision testing for the patient and simulates the standard Goldmann tet. The patient takes a seat in front of the bowl member 14 and places his head in the head positioning assembly 16. The motors 62 and 64 of the head positioning assembly 16 are actuated to move the assembly to the right, left, up and down, as required, to bring the patient's head into position so the eye being tested will be directly opposite the opening 24 in the apex 14b of the bowl 14. The light emitting diode 122 is turned on at this point and the patient will see the reflection of the diode appearing in the opening 24. The head positioning assembly 16 is moved until the patient's eye is in position so they can see the reflection of the diode. The video camera 118 is turned on also at this time to take a picture of the patient's eye. The light bulbs 67 and 128 are both turned on so that a spot is projected on to the internal surface 14a of the bowl 14, which is uniformly illuminated by the light from the bulb 128 in accordance with the standard of 31.55 APS.

The examiner will determine how many tests and what types of test strategies will be conducted on any particular patient. For example, different spot sizes may be used in combination with different colors and both kinetic and static tests can be run. When a static test is run, this spot is turned on with its intensity increasing so it reaches a threshold level which the patient can see. If a kinetic test is run, the intensity of the light is selected and the spot is moved from outside the patient's field of vision to the point where the patient will see the spot. The patient will ordinarily be more able to see light located in the center of his field of vision as opposed to the peripheral field of vision. The optical-mechanical system 12 of the present invention enables both static and kinetic test to be conducted.

When a test is run, the stepping motors are tuned on to move the spot to the desired position and then the wheels 82 and 84 are rotated to move their respective openings 86 and 90 into position allowing the spot of a given intensity and size to be projected on the internal surface 14a of the bowl 14. The use of stepping motors 160, 170 and 186 provides both a quiet and accurate way to move the spot from one location to the other in a manner which the patient will not detect these movements because the motors move the spot a distance at which corresponds to less than 1/60° of change. Such a slight movement rapidly occurring will not be detected by a patient.

## SCOPE OF THE INVENTION

The above description presents the best mode contemplated of carrying out the present invention. This invention is, however, susceptible to modifications and alternate constructions from the embodiments shown in the drawing and described above. Consequently, it is not the intention to limit this invention to the particular embodiment disclosed. On the contrary, the intention is to cover all modifications and alternate constructions within the spirit and scope of the invention as expressed by the claims.

## Claims

1. An optical-mechanical system for an automated perimeter, comprising
a hemispherically bowl member having an open front and an inside surface on which a light spot is projected;
head positioning means at the open front for holding a patient's head in position with the patient looking into the inside of the bowl member;
a light projection assembly mounted above the bowl member for projecting a spot of light onto the inside surface of the bowl member, said assembly including
a light source providing high intensity light,
a first movable mirror means comprising a member which projects downward into the bowl member and is mounted centrally at the upper front portion of the bowl member and has pivotly attached to it a mirror at the end which projects light into the bowl, said first mirror being adapted to pivot about both a horizontal and vertical axis, and a second movable mirror means comprising a pair of opposed mirrors above the bowl member and adapted to move together in unison along a horizontal path with the sum of the angle of the mirrors being equal to 90°, and
lens means disposed between the light source and the first and second mirror means and the second mirror means being disposed between the first mirror means and the lens,
said light source, lens, and first and second mirror means being in optical alignment, with the first and second mirror means being independently movable to focus and vary the size of the light spot being projected onto the inside surface of the bowl.

2. The optical-mechanical assembly of Claim 1 including filter means along the optical path to vary the brightness of the spot of light being projected onto the inside surface of the bowl member.

3. The optical-mechanical system of Claim 1 including means disposed in the optical path to vary the size of the spot of light.

4. The optical-mechanical system of Claim 1 including means disposed in the optical path for changing the color of the spot of light.

5. The optical-mechanical system of Claim 1 including means for illuminating the inside surface of the bowl in a uniform manner.

6. An optical-mechanical system for an automated perimeter, comprising
a hemispherically shaped bowl member having an open front and an inside surface on which a light spot is projected,
head positioning means mounted centrally at the open front of the bowl member for holding the patient's head in position with the patient looking into the inside of the bowl member,
a light projection assembly mounted above the bowl member for projecting light spot on to inside surface of the bowl member, said assembly including a plurality of movable mirror members and at least one lens member, said light projection assembly focusing and varying the size of the light spot projected on the inside surface of the bowl,
stepping motor means for moving the mirror means, with the light spot being simultaneously projected onto the inside surface of the bowl member, said stepping motor means moving the light spot less than 1/60 so that a patient cannot detect the stepping movement of the light spot.

7. An optical-mechanical system for an automated perimeter comprising
a hemispherical shaped bowl member having an open front and an inside white surface,
head positioning means mounted centrally at the front of the bowl for holding the patient's head in position, with the patient looking into the inside of the bowl member,
a light projection assembly mounted above the bowl member for projecting a spot of light of varying sizes, and varying light intensities, and varying colors onto the inside surface of the bowl member,
and fixation means located at the apex of the bowl to assist the patient to fixate at a central point on the inside surface of the bowl member, said fixation means including an opening in the bowl member at the apex and a light source exterior to the bowl member opposite the opening which is visible to the patient only when the patient's eye is in direct alignment with the central axis of the bowl member intersecting with the center of the opening in the bowl member.

8. The optical-mechanical system of Claim 7 wherein the fixation means includes a video camera opposite the opening at the apex for taking a picture of the eye being tested.

9. The optical-mechanical system of Claim 8 where there is a transmission mirror means disposed between the camera and the opening and the light source is adjacent hue beam splitter, with the image of the light source being reflected by the beam splitter into the bowl through the opening.

10. An optical-mechanical system for an automatic perimeter, comprising
a hemispherically shaped bowl member having an open front in an inside white surface,
head positioning means mounted centrally at the front of the bowl member for holding a patient's head in position with the patient looking into the inside of the bowl member,
a light projection assembly mounted above the bowl member for projecting light spot on the inside surface of the bowl member, and
means for providing uniform illumination of the internal surface of the bowl member comprising a light source mounted at the front lower portion of the bowl member having means for selectively distributing the amount of light over the internal surface so that the light is uniform and a lightbulb holder having an open mouth with the filament of the lightbulb just at or slightly below the open mouth.

11. An optical-mechanical system for an automatic perimeter comprising
a hemispherical shaped bowl member having an open front and an inside surface in which a spot of light is projected,
head positioning means mounted centrally at the open front of the bowl member of holding a patient's head in position with the patient looking into the inside of the bowl member,
a light projection assembly mounted above the bowl member for projecting the spot of light on t the inside surface of the bowl member, and fixation means which assists the patient in fixating on the apex of the bowl member comprising four light sources equally spaced from the apex of the bowl member at the corners of an imaginary square.

12. The optical-mechanical system of Claim 11 wherein the light source is comprised light emitting diodes disposed beneath the internal surface of the bowl member and covered with an opaque material that camoflagues these light sources when they are not emitting light but enables light to pass through this opaque barrier when the light sources are turned on.

FIG.1

FIG.2

FIG.4

FIG.3

FIG.5

FIG. 6

FIG. 7

FIG.8

FIG.9

FIG.10

FIG.11

PATIENT LINE OF SIGHT

FIG. 12

FIG. 13

FIG. 14

FIG. 15

FIG. 16

FIG. 17

*FIG. 18*

*FIG. 19*

*FIG. 20*

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB-A-1 465 561 (OCULOMETRICS INC.) * page 11, line 95 - page 12, line 31; figure 12 * | 1-3,6, 11 | A 61 B 3/02 |
| A | US-A-4 429 961 (L.A. SHEINGORN) * column 3, line 10 - column 4, line 49; figure * | 1-4,7-9 | |
| A,D | US-A-4 260 227 (C.R. MUNNERLYN et al.) * column 3, line 52 - column 7, line 33; figures 1-7 * | 1-4,6 | |

TECHNICAL FIELDS SEARCHED (Int. Cl.4)

A 61 B 3/00

The present search report has been drawn up for all claims

| Place of search | Date of completion of the search | Examiner |
|---|---|---|
| BERLIN | 25-06-1987 | WEIHS J.A. |